(19) Europäisches Patentamt
European Patent Office
Office européen des brevets

(11) Publication number: **0 012 475**
**B1**

(12) # EUROPEAN PATENT SPECIFICATION

(45) Date of publication of patent specification: **18.05.83**

(51) Int. Cl.³: **C 07 C 11/12**, C 07 C 2/74, B 01 J 31/24

(21) Application number: **79200720.5**

(22) Date of filing: **03.12.79**

(54) **A process for preparing 1,7-octadiene.**

(30) Priority: **13.12.78 US 968883**

(43) Date of publication of application:
**25.06.80 Bulletin 80/13**

(45) Publication of the grant of the patent:
**18.05.83 Bulletin 83/20**

(84) Designated Contracting States:
**DE FR GB NL**

(56) References cited:
EP - A - 0 004 408
EP - A - 0 004 409
EP - A - 0 008 139
EP - A - 0 012 472
GB - A - 1 341 324
US - A - 3 732 328
US - A - 3 823 199

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Nozaki, Kenzie**
**1407 Lakecliff**
**Houston, Texas 77077 (US)**

(74) Representative: **Puister, Antonius Tonnis, Mr. et al,**
**P.O. Box 302**
**NL-2501 CH The Hague (NL)**

(56) References cited:
**JOURNAL OF ORGANOMETALLIC CHEMISTRY, vol. 55, 16th July 1973, pages 405—407, Lausanne, CH, P. ROFFIA et al.: "Catalysis by palladium salts. IV. Selective hydrogenation with formic acid in the palladium catalysed dimerisation of 1,3-butadiene; syntheses of 1,7-octadiene"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 012 475 B1

# A process for preparing 1,7-octadiene

The present invention is concerned with a process for preparing 1,7-octadiene by hydrodimerizing butadiene in the presence of a catalytic amount of palladium and a tertiary organo phosphorus ligand, formic acid or a salt thereof and optionally a solvent.

Hydrodimerizing butadiene (1,3-butadiene) with formic acid and palladium catalyst is known; e.g. U.S. Patent 3,732,328 discloses the preparation of mixtures of octadienes by reacting butadiene in the presence of a palladium compound, a polar solvent, a reducing agent and a tertiary phosphine. In addition, U.S. Patent 3,823,199 discloses the preparation of mixtures of octadiene by reacting butadienes in the presence of a palladium compound, a non-polar solvent, a reducing agent and a tertiary phosphine. Moreover, U.K. Patent 1,341,324 discloses processes similar to above. Furthermore, Tetrahedron Letters No. 2, 1972, pages 163—164 discloses the production of mixtures of octadiene by reacting butadiene in the presence of palladium salts, or organic base, formic acid and a phosphine and the Journal of Organometallic Chemistry, 55 (1973), pages 405—507 utilizes a phophine-zero valent palladium complex catalyst in benzene in the presence of formic acid to dimerize butadiene.

The Applicants have now found that the utilization of mixtures of different tertiary organo phosphine compounds results in enhanced catalyst activity.

According to the present invention a process for preparing 1,7-octadiene comprising hydrodimerizing butadiene in the presence of a catalytic amount of palladium and a tertiary organo phosphorus ligand, formic acid or a salt thereof and optionally a solvent, is characterized in that a mixture of ligands is present comprising a first ligand selected from the group consisting of tertiary organo phophines, phosphinites and phosphonites represented by the formula $(RO)_aPR_b$, wherein a+b equals 3, a is 0, 1 or 2, and at least one R is a sterically hindered group being a branched alkyl group or an aralkyl, alkenyl or cycloalkyl group, the aforementioned groups having from 3 to 10 carbon atoms whilst branching occurring in alkyl groups is at a carbon atom no more than two carbon atoms from the phosphorus atom, and at least one other ligand selected from the group consisting of tertiary organo phosphines, phosphinites, phosphonites, and phosphites represented by the formula $(RO)_{a'}PR_{b'}$, wherein R is an organic group and wherein a'+b' equals 3, a' is 0, 1, 2 or 3, and a' is not the same as a.

Accordingly, one of the ligands utilized according to the invention is a sterically hindered phosphine, phosphinite or phosphonite and the other ligand is phosphine, phosphinite, phosphonite or phosphite which may or may not be sterically hindered, preferably not sterically hindered and is preferably selected from a different class. This selection of ligands provides a synergistic improvement in catalytic activity.

For the sake of completeness reference is made to EP Applications 79 200 141.4, 79 200 392.3 and 79 200 140.6, in which processes for making 1,7-octadiene are described which are similar to the presently proposed one except that they do not utilize a mixture of different ligands.

As indicated above, it is not essential to use solvents in the process of this invention but a suitably selected solvent can promote the rate of reaction by a factor of two or more.

The non-polar solvents disclosed in U.S. Patent 3,823,199, such as paraffinic, cycloparaffinic or aromatic solvents are useful in the process of this invention. The solvent can be a paraffin or cycloparaffin containing 5 to 16 carbon atoms, and the like. Suitable solvents also include aromatic hydrocarbons such as benzene, lower alkyl-substituted aromatic hydrocarbons, such as toluene, m-, p- and o-xylene, halogenated aromatic hydrocarbons including chloro, bromo and iodo substituted, such as chlorobenzene and the like. Halogenated lower aliphatic compounds, such as chloroform, methylene chloride, carbon tetrachloride and the like may be used, in particular chloroform is preferred.

Further useful solvents are the amine solvents disclosed in U.K. Patent 1,341,324. A wide range of amines are useful provided that they are liquid under reaction conditions. Tertiary amines are preferred to primary and secondary amines. Suitable amine solvents include alkylamines, cycloalkylamines, arylamines and heterocyclic amines, such as morpholine, pyridine, piperazine and piperidine. Examples of these classes of amines are the lower alkylamines containing 2 to 6 carbon atoms in each alkyl group, such as triethylamine; mono-cyclohexylamine, and N-alkylcyclohexylamines containing up to 12 carbon atoms; aniline and N-alkylanilines containing up to 12 carbon atoms and N-alkylmorpholines containing up to 12 carbon atoms. The preferred amine solvent is pyridine.

Further useful solvents are those of moderate coordinating ability, and which include nitriles such as lower alkyl nitriles, hydrocarbon aromatic nitriles including acetonitrile, benzonitrile and the like, amides including benzamide, acetamide, mono- and di-substituted amides where the substituent is preferably lower alkyl. Suitable substituted amides include N-methyl acetamide, N,N-dimethyl acetamide and dimethylformamide. Dialkyl sulphoxides, such as dimethyl sulphoxide and sulphones, such as sulfolane and alkyl-substituted sulfolane are satisfactory. By dialkyl it is meant that the sulphur and nitrogen atoms are connected to two different carbon atoms. They may be separate alkyl groups or the same, i.e., a ring alkyl group, e.g., tetramethylene sulphoxide and N-methyl pyrrolidinone. The alkyl moieties have carbon numbers ranging from 1 to about 6. Simple ethers, such as the dilower alkyl ethers including dimethyl ether, diethylene, and the like, function satisfactorily. Hydrocarbon aromatic

ethers, such as the lower alkyl phenyl ethers, may be also used. In addition, the cyclic diethers, such as 1,4-dioxane, are also suitable solvents.

Simple lower alkyl esters of lower alkanoic acids, such as ethyl acetate, methyl acetate, methyl butyrate and the like as well as cyclic diesters, such as ethylene carbonate, are also suitable solvents of moderate coordinating ability. Ketones, including lower aliphatic ketones, such as methyl ethyl ketone and hydrocarbon aromatic ketones, such as acetophenone are also satisfactory solvents. Lower mono- and di-alkanols, such as isopropanol, ethylene glycol and the like may be used, if desired. The preferred solvents of moderate coordinating ability include nitriles, formamides, such as dimethylformamide, dilower alkyl ethers, lower alkyl phenyl ethers, simple lower alkyl esters of lower alkanoic acids, ketones and lower alkanols.

Particularly useful solvents include pyridine, benzene, dimethylformamide, dimethylsulphoxide, chlorobenzene, anisol, N,N-dimethylacetamide, nitromethane, ethyl acetate, isopropanol, benzonitrile, chloroform, methyl ether ketone, acetonitrile, diethylether, acetopheneneon, toluene, ethylene glycol, ethyl carbonate, propylene carbonate and sulfolane. Particularly desired solvents are pyridine, nitromethane, dimethylsulphoxide, ethylene carbonate and propylene carbonate.

The preferred organic solvents will have carbon numbers ranging from 1 to about 20. Particularly desired solvents are those which give two-phase systems which allow easy product separation such as, for example, nitromethane, ethylene carbonate and propylene carbonate.

The amount of solvent added should be sufficient to dissolve the palladium compound-tertiary organo phosphorus complex.

The formic acid is utilized as a source of hydrogen for the process. It is present in the reaction mixture as an acid or as a salt of a base. When the salt is used, it is thought that dissociation of the formic acid-base salt provides a suitable amount of formic acid necessary to provide the required hydrogen.

It is desirable that some formic acid or the salt, be present during the entire course of the reaction. When operating the process batch-wise, this can be accomplished by adding a stoichiometric amount of formic acid initially, 1 mol. of formic acid for every 2 moles of butadiene, or by continuously or periodically adding additional amounts of formic acid.

A base when used must be one which can neutralize the formic acid according to the reaction:

$$HCOOH+B\rightarrow HCOO^{-}HB^{+}.$$

The base may be organic or inorganic. Suitable organic bases typically have dissociation constants greater than $10^{-8}$ and include tertiary amines such as triethyl amine, tributyl amine, dimethylethyl amine, lutidine, tripropyl amine, N-methyl morpholine, isoquinoline, N-methyl-2,2,6,6-tetramethyl piperidine, 2,8-(dimethylamine)naphthalene and the like.

Suitable inorganic bases include ammonia, the hydroxide bases, such as sodium hydroxide, potassium hydroxide, calcium hydroxide; ammonium hydroxide; the carbonates and bicarbonates such as sodium carbonate, sodium bicarbonate, potassium carbonate, potassium bicarbonate, calcium carbonate and the like; the weak bases, such as sodium acetate, potassium acetate, ammonium carbonate, ammonium acetate and the like. When the inorganic bases are utilized, small amounts of water may be present. Preferred moles of water are at least equal to the moles of formate salts.

When organic bases are utilized, excess base may be utilized as a solvent or the amine-base salt may be used as the solvent.

The catalyst used in the process of this invention is palladium or a palladium compound complexed with a tris-organo phosphorus-containing ligand. The palladium may be in any of its possible valence states, e.g., 0, +2, etc. Suitable palladium compounds include the palladium carboxylates, particularly palladium carboxylates derived from alkanoic acids containing up to six carbon atoms, such as palladium acetate (OAC), complexes such as palladium acetylacetonate (AcAc), bis-benzonitrile palladium(II) and lithium palladous chloride as well as the palladium halides, nitrates and sulphates, such as palladous chloride and palladium nitrate $Pd(NO_3)_2(OH)_2$ and palladium sulphate. The palladium is present in the reaction mixture in catalytic amounts; preferably from 1 to $10^{-6}$ molar and more preferably from $10^{-1}$ to $10^{-4}$ molar.

The palladium compounds complexed with a tris-organo phosphorus-containing ligand are typically prepared by reacting the tertiary phosphorus ligand with the appropriate palladium compound as, for example, represented by the following equations:

$$(RO)_aPR_b+(RO)_{a'}PR_{b'}+(PHCN)_2PdCl_2\rightarrow[(RO)_aPR_b][(RO)_{a'}PR_{b'}]PdCl_2$$

$$[(RO)_aPR_b][(RO)_{a'}PR_{b'}]PdCl_2+Ag_2Co_3\rightarrow[(RO)_aPR_b][(RO)_{a'}PR_{b'}]PdCO_3$$

$$[(RO)_aPR_b][(RO)_{a'}PR_{b'}]PdO_2+SO_2\rightarrow[(RO)_aPR_b][(RO)_{a'}PR_{b'}]PdSO_4$$

$$[(RO)_aPR_b][(RO)_{a'}PR_{b'}]PdO_2+N_2O_4\rightarrow[(RO)_aPR_b][(RO)_{a'}PR_{b'}]Pd(NO_3)_2$$

where $[(RO)_aPR_b][(RO)_{a'}PR_{b'}]$ represents the mixture of ligands of the present invention and R, a, b, a' and b' are as defined below.

The ligands used in the present invention will comprise a mixture of two ligands which provide a synergistic improvement in catalytic activity over the use of the individual ligands. A first ligand, preferably present in the amount of at least one mole of ligand per mole of palladium, is a sterically hindered tertiary organo phosphorus compound selected from one of the following three classes of tertiary organo phosphorus compounds: phosphines, phosphinites and phosphonites; which can be represented by the following general formula:

$$(RO)_aPR_b \qquad (I)$$

where a+b equals 3; a is 0, 1 or 2 and R generally is the same or different organic group, such as a hydrocarbyl group, and preferably is an aryl, alkyl, alkenyl, cycloalkyl, aralkyl or alkaryl group containing not more than about 20 carbon atoms, preferably not more than 12 carbon atoms, with at least one of the R groups being a sterically hindering group, such as a branched alkyl, or an aralkyl, alkenyl and cycloalkyl having from 3 to 10 carbon atoms with branching occurring at a carbon atom not more than two carbon atoms from the phosphorus atom. Suitable branched groups are those of which the alpha- and beta-carbon atoms, relative to the phosphorus atom, are not both $-CH_2$-linkages.

Suitable examples of R in general are phenyl, p-tolyl, o-tolyl, m-tolyl, m-chlorophenyl, p-chlorophenyl, p-anisoyl, m-anisoyl, methyl, ethyl, propyl, butyl and the like. It is also suitable for the organic group R to contain functional groups or to satisfy more than one of the valences of the phosphorus atom, thereby forming a heterocyclic compound with the phosphorus atom. Preferably R represents an aryl, alkyl, alkenyl, cycloalkyl, aralkyl or an alkaryl group or a mixture thereof having carbon numbers from 1 to 20, preferably 1 to 12 carbon atoms and need not be the same, e.g., $R^1R^2R^3P$, $(R^1O)PR^2R^3$, $(R^1O)(R^2O)PR^3$, $R^1PR^2$. Particularly suitable non-sterically hindering R groups include methyl, ethyl, n-propyl, n-butyl, phenyl and chlorophenyl groups.

Illustrative of the sterically hindering R group are, for alkyl, isopropyl, secondary butyl, tertiary butyl, isobutyl, neopentyl, secondary pentyl, tertiary pentyl, 2-methylbutyl, secondary hexyl, tertiary hexyl, 2,2-dimethylpropyl; for aralkyl, benzyl, alpha-methylbenzyl, alpha,alpha-dimethylbenzyl, alphamethyl-alpha-ethylbenzyl, phenylethyl, phenylisopropyl, phenyl-tertiary-butyl; for alkenyl, allyl, crotyl, methallyl, 1-methylethenyl, 1-methyl-2-propenyl, 2-methyl-2-propenyl, 1,1-dimethyl-2-propenyl, 1-methyl-3-butenyl and for cycloalkyl, cyclopropyl, cyclobutyl, cyclopentyl, cyclohexyl, cycloheptyl, and the like. Particularly suitable sterically hindering R groups include benzyl, isopropyl, isobutyl, secondary butyl, tertiary butyl and cyclohexyl groups.

Alternatively the formula for the first ligand can be expressed as:

$$R^1_cR^2_dR^3_eP(OR^4)_f(OR^5)_g \qquad (II)$$

where c, d, e, f and g individually equals 0 or 1, c+d+e+f+g equals 3 and R is as defined above, $R^1$, $R^2$, $R^3$, $R^4$ and $R^5$ may be the same or different. Again, it must be emphasized, that at least one of the R groups must be a sterically hindering group and is selected from a branched alkyl, or an aralkyl, alkenyl, and cycloalkyl having from 3 to about 10 carbon atoms with branching occurring at a carbon atom not more than two carbon atoms from the phosphorus atom. This R group provides a steric hindrance to the catalyst complex which enhances selectivity.

A second ligand preferably present in the amount of at least one mole of ligand per mole of palladium is a tertiary organo phosphorus compound of a different class than the first ligand and is selected from one of the following classes of tertiary organic phosphorus compounds: phosphines, phosphinites, phosphonites and phosphites. This second ligand can be represented by the following general formula:

$$(RO)_{a'}PR_{b'} \qquad (III)$$

where a'+b' equals 3, a' is 0, 1, 2 or 3, a' is not the same as a above and R generally is the same or different organic group, such as a hydrocarbyl group and preferably is an aryl, alkyl, alkenyl, cycloalkyl, aralkyl or alkaryl group containing not more than 20 carbon atoms, preferably not more than 12 carbon atoms.

Suitable examples of R are phenyl, p-tolyl, o-tolyl, m-tolyl, m-chlorophenyl, p-chlorophenyl, p-anisoyl, m-anisoyl, methyl, ethyl, propyl, butyl and the like. It is also suitable for the organic radical R to contain functional groups or to satisfy more than one of the valences of the phosphorus atoms, thereby forming a heterocyclic compound with the phosphorus atom. Preferably R represents an aryl, alkyl, aralkyl or alkaryl group or a mixture thereof having carbon numbers from 1 to 20, preferably 1 to 12 carbon atoms and need not be the same, e.g. $R^1R^2R^3P$, $(R^1O)PR^2R^3$, $(R^1O)(R^1O)PR^3$, $R^1PR^2$. Particularly suitable groups include methyl, ethyl, n-propyl, n-butyl, phenyl and chlorophenyl groups.

Alternatively, the formula from the second ligand can be expressed as:

$$R^1_hR^2_iR^3_jP(OR^4)_k(OR^5)_l(OR^6)_m \qquad (IV)$$

where h, i, j, k, l and m are individually 0 or 1, h+i+j+k+l+m equals 3 and R is as defined generally

above. $R^1$, $R^2$, $R^3$, $R^4$, $R^5$ and $R^6$ may be the same or different. The second ligand may or may not have sterically hindering groups incorporated therein. If sterically hindered groups are present then particularly suitable groups are those described above for formula (I).

The mole ratio of tertiary phosphorus ligand to palladium is preferably at least 2. More preferably the mole ratio of ligand to palladium ranges from about 2:1 to about 20:1 and most preferably from about 2:1 to about 5:1. The use of the tertiary phosphorus ligands of the invention provide extremely high selectivities to 1,7-octadiene as well as high activity for conversion of butadiene.

Alternatively, the palladium compound and the tertiary phosphorus ligand may be bound onto a cross-linked synthetic resin instead of being dissolved in the reaction medium. Suitable cross-linked synthetic resins include cross-linked polystyrene, poly(alpha-alkyl)acrylates, polycarbonates, polyamides and the like. In general, one of the ligands will be bound to the resin. If the first ligand is bound to the cross-linked synthetic resin then it may be represented by the general formula $Z—P(OR)_nR_p$, wherein n is 0, 1 or 2 and p+n equals 2 and the second ligand by the general formula $(RO)_{a'}PR_{b'}$, wherein R, a' and b' are as herein defined, with the first ligand having at least one sterically hindering group as herein defined. If the second ligand is bound to the cross-linked synthetic resin then it may also be represented by the general formula $Z—P(OR)_nR_p$ and the first ligand by the general formula $(RO)_aPR_b$, wherein R, n, p, a and b are as herein defined; in this case the ligand bound to the cross-linked synthetic resin may or may not have sterically hindering groups. In these cases the molar ratios referred to above are on a phosphorus basis.

The bound tertiary phosphine may also be represented by the general formula:

$$\begin{array}{c} (R_6)_q—(R_6)_r \\ | \\ (RO)_n—P—R_p \end{array} \qquad (V)$$

wherein R, n and p are defined previously, and $R_6$ represents the repeating unit of the synthetic resin and where q is a positive integer, r is 0 or a positive integer, q+r equals the total number of repeating units in resin and the percentage of the repeating units substituted with the tertiary phosphine is represented by the formula:

$$\frac{8q}{q+r} \times 100\%. \qquad (VI)$$

The number of repeating units substituted with the tertiary phosphine is not critical. When less than 5% of the repeating units contain a phosphine substitute, large quantities of the resin must be used to form the bound catalyst. Accordingly, it is desirable to have at least 10% of the repeating units substituted with a tertiary phosphine. It is preferred, however, that from 20 to 40% of the repeating units contain a phosphine substituent. The substituent can be introduced into the resin using well-known techniques, such as those described in the Journal of the American Chemical Society, *97* (7) 1749 (1975) and in Ann. N. Y. Academy of Sciences, *239*, 76 (1974). In accordance with those techniques, the palladium compound is complexed with the phosphorus-substituted resin by admixing in a solvent for the palladium compound.

The catalyst may be pretreated to enhance reactivity by contacting it with a reducing agent at a temperature of from about 20 to about 90°C for from about 0.1 to about 5 hours. The reducing agent may be gaseous, solid or liquid. Examples of gaseous agents are hydrogen, and carbon monoxide. Examples of liquid or solid reducing agents are hydrazine, $NaBH_4$, $NaOCH_3$, $(isopropyl)_3P$, Cu, Na and Al alkyls, etc. The reduction may be carried out in a separate autoclave or preferably is carried out in the hydrodimerization reactor prior to the introduction of the butadiene. The palladium compound-tris-organo phosphorus complex may be dissolved in the solvent used in this invention prior to reduction.

The process can be either continuous or batch. The reaction temperature of the process is not critical, however, it is preferred to maintain the reaction temperature in the range of from 0 to about 100°C preferably from about 20 to about 70°C. The process is conducted under a sufficient pressure to maintain liquid phase conditions at the reaction temperature. Typically the pressure is autogenous.

The process of this invention is particularly useful when a butadiene(isobutene)n-butenes (BBB) stream from an oil pyrolysis unit is utilized to provide the butadiene. These BBB streams are the $C_4$ cut from a thermal cracking unit typically containing 30—40% butadiene, 20—35% isobutene and 20—30% n-butenes and many minor components.

The invention will now be illustrated by reference to the following Examples.

Examples 1 to 104

To an 80 ml glass-lined autoclave were charged $2.7 \times 10^{-5}$ moles of palladium as a 10% water solution of $Pd(NO_3)_2(OH)_2$, 10 ml of pyridine, 2 g of butadiene, $1.85 \times 10^{-2}$ moles of formic acid salt ($Et_3N$ . HOOCH) and sufficient phosphorus ligand to provide the appropriate ligand to palladium ratio as shown in Table I below. The stirred reactor was heated to 40°C for two hours, cooled and the product was analyzed by gas chromatography and mass spectrography. The results are shown in the following Table.

TABLE I

| Example | Phosphite | P/Pd ratio | Phosphine | P/Pd ratio | Butadiene conversion mol. % | 1,7-Octadiene selectivity mol. % |
|---|---|---|---|---|---|---|
| 1 | — | — | P(isopropyl)$_3$ | 1 | 21.0 | 95.5 |
| 2 | — | — | P(isopropyl)$_3$ | 2 | 30.0 | 98.0 |
| 3 | P(O CH$_3$)$_3$ | 1 | — | — | 8.5 | 71.0 |
| 4 | P(O CH$_3$)$_3$ | 2 | — | — | 10.0 | 72.0 |
| 5 | P(O Ethyl)$_3$ | 1 | — | — | 5.2 | 67.0 |
| 6 | P(O Ethyl)$_3$ | 2 | — | — | 11.0 | 78.0 |
| 7 | P(O isopropyl)$_3$ | 1 | — | — | 5.5 | 59.0 |
| 8 | P(O isopropyl)$_3$ | 2 | — | — | 16.0 | 76.0 |
| 9 | P(O n-butyl)$_3$ | 2 | — | — | 1.8 | 80.0 |
| 10 | P(O phenyl)$_3$ | 1 | — | — | 1.7 | 75.0 |
| 11 | P(O phenyl)$_3$ | 2 | — | — | 2.0 | 75.0 |
| 12 | P(O p-ClØ)$_3$ | 2 | — | — | 0.5 | low |
| 13 | P(OCH$_3$)$_3$ | 1 | P(isopropyl)$_3$ | 1 | 48.0 | 95.0 |
| 14 | P(OCH$_3$)$_3$ | 1 | P(isopropyl)$_3$ | 2 | 91.0 | 98.1 |
| 15 | P(OCH$_3$)$_3$ | 2 | P(isopropyl)$_3$ | 2 | 77.0 | 98.3 |
| 16 | P(O Ethyl)$_3$ | 1 | P(isopropyl)$_3$ | 2 | 84.0 | 99.0 |
| 17 | P(O ethyl)$_3$ | 2 | P(isopropyl)$_3$ | 2 | 88.0 | 96.5 |
| 18 | P(O isopropyl)$_3$ | 1 | P(isopropyl)$_3$ | 1 | 56.0 | 96.5 |
| 19 | P(O isopropyl)$_3$ | 1 | P(isopropyl)$_3$ | 2 | 85.0 | 99.0 |
| 20 | P(O isopropyl)$_3$ | 2 | P(isopropyl)$_3$ | 1 | 47.0 | 92.5 |
| 21 | P(O isopropyl)$_3$ | 2 | P(isopropyl)$_3$ | 2 | 98.0 | 98.5 |
| 22 | P(O n-butyl)$_3$ | 1 | P(isopropyl)$_3$ | 1 | 44.0 | 95.0 |
| 23 | P(O n-butyl)$_3$ | 1 | P(isopropyl)$_3$ | 2 | 82.0 | 98.5 |
| 24 | P(O n-butyl)$_3$ | 2 | P(isopropyl)$_3$ | 2 | 82.0 | 98.3 |
| 25 | P(O phenyl)$_3$ | 1 | P(isopropyl)$_3$ | 1 | 11.0 | 98.0 |
| 26 | P(O phenyl)$_3$ | 2 | P(isopropyl)$_3$ | 2 | 23.0 | 98.0 |
| 27 | P(O p-ClØ)$_3$ | 2 | P(isopropyl)$_3$ | 2 | 52.0 | 95.3 |
| 28 | P(O isopropyl)$_3$ | 1 | P(n-butyl)$_3$ | 1 | 17.0 | 79.0 |
| 29 | P(O isopropyl)$_3$ | 2 | P(n-butyl)$_3$ | 2 | 37.0 | 85.0 |
| 30 | P(O isopropyl)$_3$ | 1 | P(phenyl)$_3$ | 1 | 9.4 | 85.0 |
| 31 | P(O isopropyl)$_3$ | 2 | P(phenyl)$_3$ | 2 | 19.0 | 87.0 |
| 32 | P(O isopropyl)$_3$ | 1 | P(ethyl)$_3$ | 1 | 14.0 | 77.0 |
| 33 | — | — | P(cyclohexyl)$_3$ | 1 | 25.0 | 97.0 |
| 34 | — | — | P(cyclohexyl)$_3$ | 2 | 40.0 | 98.0 |
| 35 | P(O ethyl)$_3$ | 1 | P(cyclohexyl)$_3$ | 2 | 62.0 | 97.0 |
| 36 | P(O isopropyl)$_3$ | 1 | P(cyclohexyl)$_3$ | 2 | 61.0 | 97.8 |
| 37 | P(O isopropyl)$_3$ | 2 | P(cyclohexyl)$_3$ | 2 | 65.0 | 96.0 |
| 38 | — | — | P(sec. butyl)$_3$ | 2 | 25.0 | 97.5 |
| 39 | P(O isopropyl)$_3$ | 2 | P(sec. butyl)$_3$ | 2 | 95.0 | 97.8 |
| 40 | — | — | P(t-butyl)$_2$ benzyl | 2 | 23.0 | 99.2 |
| 41 | P(O isopropyl)$_3$ | 2 | P(t-butyl)$_2$ benzyl | 2 | 82.0 | 98.2 |
| 42 | P phenyl (O n-butyl)$_2$ | 2 | — | — | 1.4 | 75.0 |
| 43 | P phenyl (O n-butyl)$_2$ | 1 | P(isopropyl)$_3$ | 2 | 73.0 | 99.0 |
| 44 | P phenyl (O n-butyl)$_2$ | 2 | P(isopropyl)$_3$ | 2 | 80.0 | 98.6 |
| 45 | P t.-butyl (OCH$_3$)$_2$ | 2 | — | — | 35.0 | 93.6 |
| 46 | P t.-butyl (OCH$_3$)$_2$ | 2 | P(isopropyl)$_3$ | 2 | 95.0 | 98.0 |
| 47 | P t.-butyl (OCH$_3$)$_2$ | 4 | P(isopropyl)$_3$ | 2 | 80.0 | 98.6 |
| 48 | P t.-butyl (O benzyl)$_2$ | 2 | — | — | 26.0 | 94.0 |
| 49 | P t.-butyl (O benzyl)$_2$ | 2 | P(isopropyl)$_3$ | 2 | 72.0 | 98.3 |
| 50 | P t.-butyl (O benzyl)$_2$ | 1 | P(isopropyl)$_3$ | 1 | 59.0 | 97.8 |
| 51 | P t.-butyl (O isopropyl)$_2$ | 2 | — | — | 60.0 | 97.3 |
| 52 | P t.-butyl (O isopropyl)$_2$ | 1 | — | — | 34.0 | 96.0 |
| 53 | P t.-butyl (O isopropyl)$_2$ | 1 | P(isopropyl)$_3$ | 1 | 42.0 | 97.0 |
| 54 | P t.-butyl (O isopropyl)$_2$ | 1 | P(isopropyl)$_3$ | 2 | 82.0 | 98.6 |
| | Phosphinite | | | | | |
| 55 | P(phenyl)$_2$ O n-butyl | 2 | — | — | 12.0 | 74.0 |
| 56 | P(phenyl)$_2$ O n-butyl | 2 | P(isopropyl)$_3$ | 2 | 86.0 | 98.5 |
| 57 | P(cyclohexyl)$_2$OCH$_3$ | 2 | — | — | 18.0 | 96.5 |
| 58 | P(cyclohexyl)$_2$OCH$_3$ | 2 | P(isopropyl)$_3$ | 2 | 60.0 | 98.0 |

TABLE I (cont.)

| Example | Phosphite | P/Pd ratio | Phosphine | P/Pd ratio | Butadiene conversion mol. % | 1,7-Octadiene selectivity mol. % |
|---|---|---|---|---|---|---|
| 59 | P(t-butyl)$_2$ O benzyl | 1 | — | — | 31.0 | 98.6 |
| 60 | P(t-butyl)$_2$ O benzyl | 2 | — | — | 39.0 | 98.8 |
| 61 | P(t-butyl)$_2$ O benzyl | 4 | — | — | 51.0 | 99.3 |
| 62 | P(t-butyl)$_2$ O benzyl | 1 | P(isopropyl)$_3$ | 1 | 47.0 | 98.9 |
| 63 | P(t-butyl)$_2$ O benzyl | 2 | P(isopropyl)$_3$ | 2 | 61.0 | 99.3 |
| 64 | P(cyclohexyl)$_2$ O cyclohexyl | 1 | — | — | 55.0 | 95.3 |
| 65 | P(cyclohexyl)$_2$ O cyclohexyl | 2 | — | — | 80.0 | 98.0 |
| 66 | P(cyclohexyl)$_2$ O cyclohexyl | 1 | P(isopropyl)$_3$ | 1 | 97.0 | 98.1 |
| 67 | P(cyclohexyl)$_2$ O cyclohexyl | 2 | P(isopropyl)$_3$ | 2 | 98.5 | 99.0 |
| 68 | — | — | P(phenyl)$_3$ | 2 | 8.4 | 87.0 |
| 69 | P(t-butyl)$_2$ O benzyl | 1 | P(phenyl)$_3$ | 1 | 30.0 | 91.3 |
| 70 | — | — | P(n-butyl)$_3$ | 2 | 21.0 | 89.0 |
| 71 | P(t-butyl)$_2$ O benzyl | 1 | P(n-butyl)$_3$ | 1 | 56.0 | 98.0 |
| | **Phosphites** | | **Phosphinites** | | | |
| 72 | — | — | P(t-butyl)$_2$ O benzyl | 1 | 31.0 | 98.6 |
| 73 | — | — | P(t-butyl)$_2$ O benzyl | 2 | 39.0 | 98.8 |
| 74 | — | — | P(t-butyl)$_2$ O benzyl | 4 | 51.0 | 99.3 |
| 75 | P(OCH$_3$)$_3$ | 1 | — | — | 8.5 | 71.0 |
| 76 | P(OCH$_3$)$_3$ | 1 | P(t-butyl)$_2$ O benzyl | 1 | 80.0 | 97.8 |
| 77 | P(O ethyl)$_3$ | 1 | — | — | 5.2 | 67.0 |
| 78 | P(O ethyl)$_3$ | 1 | P(t-butyl)$_2$ O benzyl | 1 | 78.0 | 98.3 |
| 79 | P(O isopropyl)$_3$ | 1 | — | — | 5.5 | 59.0 |
| 80 | P(O isopropyl)$_3$ | 1 | P(t-butyl)$_2$ O benzyl | 1 | 80.0 | 98.5 |
| 81 | P(O n-butyl)$_3$ | 1 | — | — | 15.5 | 56.0 |
| 82 | P(O n-butyl)$_3$ | 1 | P(t-butyl)$_2$ O benzyl | 1 | 78.0 | 97.8 |
| 83 | P(O phenyl)$_3$ | 1 | — | — | 1.7 | 75.0 |
| 84 | P(O phenyl)$_3$ | 1 | P(t-butyl)$_2$ O benzyl | 1 | 26.0 | 98.0 |
| 85 | — | — | P(cyclohexyl)$_2$ O cyclohexyl | 1 | 55.0 | 95.3 |
| 86 | — | — | P(cyclohexyl)$_2$ O cyclohexyl | 2 | 80.0 | 98.0 |
| 87 | P(O isopropyl)$_3$ | 1 | P(cyclohexyl)$_2$ O cyclohexyl | 1 | 89.0 | 99.2 |
| 88 | P(n-butyl)$_3$ | 1 | P(cyclohexyl)$_2$ O cyclohexyl | 1 | 43.0 | 95.0 |
| | **Phosphites** | | **Phosphonites** | | | |
| 89 | — | — | Pt-butyl (O benzyl)$_2$ | 1 | 15.3 | 92.2 |
| 90 | — | — | Pt-butyl (O benzyl)$_2$ | 2 | 26.0 | 94.0 |
| 91 | P(OCH$_3$)$_3$ | 1 | Pt-butyl (O benzyl)$_2$ | 1 | 18.7 | 86.0 |
| 92 | P(O isopropyl)$_3$ | 1 | Pt-butyl (O benzyl)$_2$ | 1 | 27.0 | 87.0 |
| 93 | — | — | Pt-butyl (O propyl)$_2$ | 1 | 34.0 | 96.0 |
| 94 | P(O ethyl)$_3$ | 1 | Pt-butyl (O propyl)$_2$ | 1 | 37.0 | 94.0 |
| 95 | P(O isopropyl)$_3$ | 1 | Pt-butyl (O propyl)$_2$ | 1 | 42.0 | 97.0 |
| 96 | P(O phenyl)$_3$ | 2 | Pt-butyl (O propyl)$_2$ | 1 | 20.0 | 96.4 |
| 97 | P(t-butyl) (O benzyl)$_2$ | 1 | — | — | 15.3 | 92.2 |
| 98 | P(t-butyl) (O benzyl)$_2$ | 2 | — | — | 26.0 | 94.0 |
| 99 | — | — | P(t-butyl)$_2$ O benzyl | 1 | 31.0 | 98.6 |
| 100 | — | — | P(t-butyl)$_2$ O benzyl | 2 | 39.0 | 98.8 |
| 101 | P(t-butyl) (O benzyl)$_2$ | 1 | P(t-butyl)$_2$ O benzyl | 1 | 73.0 | 98.3 |
| 102 | — | — | P(cyclohexyl)$_2$ O cyclohexyl | 1 | 55.0 | 95.3 |
| 103 | — | — | P(cyclohexyl)$_2$ O cyclohexyl | 2 | 80.0 | 98.0 |
| 104 | P(t-butyl) (O benzyl)$_2$ | — | P(cyclohexyl)$_2$ O cyclohexyl | 1 | 76.0 | 92.0 |

Examples 105 and 106

To an 80 ml glass-lined autoclave were charged $2.7 \times 10^{-5}$ moles of palladium acetylacetonate, 10 ml of dimethylsulphoxide, 2 g of butadiene, $1.85 \times 10^{-2}$ moles of formic acid and sufficient phosphorus ligand to provide the appropriate ligand to palladium ratio as shown in Table II below. The stirred reactor was heated to 40°C for two hours, cooled and the product was analyzed by gas chromatography and mass spectrometry. The results are shown in Table II.

TABLE II

| Example | Phosphine | P/Pd ratio | Phosphite | P/Pd ratio | Butadiene conversion mol. % | 1,7-Octadiene selectivity mol. % |
|---|---|---|---|---|---|---|
| 105 | $P(sec\text{-}butyl)_3$ | 2 | — | — | 20.0 | 98.5 |
| 106 | $P(sec\text{-}butyl)_3$ | 2 | $P(O\text{-}ethyl)_3$ | 2 | 85.0 | 98.0 |

Examples 107 to 109

To an 80 ml glass-lined autoclave were charged $2.7 \times 10^{-5}$ moles of palladium acetylacetonate, 10 ml of dimethylsulphoxide, 2 g of butadiene, $1.85 \times 10^{-2}$ moles of sodium formate, 1 ml of $H_2O$ and sufficient phosphorus ligand to provide the appropriate ligand to palladium ratio as shown in Table III below. The stirred reactor was heated to 40°C for two hours, cooled and the product was analyzed by gas chromatography and mass spectrometry. The results are shown in Table III.

TABLE III

| Example | Phosphine | P/Pd ratio | Phosphite | P/Pd ratio | Butadiene conversion mol. % | 1,7-Octadiene selectivity mol. % |
|---|---|---|---|---|---|---|
| 107 | $P(isopropyl)_3$ | 2 | — | — | 27.0 | 97.0 |
| 108 | — | — | $P(O\text{-}isopropyl)_3$ | 2 | 13.0 | 93.0 |
| 109 | $P(isopropyl)_3$ | 2 | $P(O\text{-}isopropyl)_3$ | 2 | 54.0 | 97.3 |

**Claims**

1. A process for preparing 1,7-octadiene comprising hydrodimerizing butadiene in the presence of a catalytic amount of palladium and a tertiary organo phosphorus ligand, formic acid or a salt thereof and optionally a solvent, characterized in that a mixture of ligands is present comprising a first ligand selected from the group consisting of tertiary organo phosphines, phosphinites and phosphonites represented by the formula $(RO)_aPR_b$, wherein a+b equals 3, a is 0, 1 or 2 and at least one R is a sterically hindered group being a branched alkyl group or an aralkyl, alkenyl or cycloalkyl group, the aforementioned groups having from 3 to 10 carbon atoms whilst branching occurring in alkyl groups is at a carbon atom no more than two carbon atoms from the phosphorus atom, and at least one other ligand selected from the group consisting of tertiary organo phosphines, phosphinites, phosphonites and phosphites represented by the formula $(RO)_{a'}PR_{b'}$, wherein R is an organic group and wherein a'+b' equals 3, a' is 0, 1, 2 or 3, and a' is not the same as a.

2. A process as claimed in claim 1, characterized in that the organic groups are aryl, alkyl, alkenyl, cycloalkyl, aralkyl or alkaryl groups containing no more than 20 carbon atoms.

3. A process as claimed in claim 1, characterized in that one of the organic groups of the first or second ligand is a cross-linked synthetic resin.

4. A process as claimed in claim 3, characterized in that the resin is cross-linked polystyrene, poly(alpha-alkyl)acrylate, polycarbonate or polyamide.

5. A process as claimed in any one of claims 1 to 4, characterized in that the amount of palladium is from about 1 to about $10^{-6}$ molar.

6. A process as claimed in any one of claims 1 to 5, characterized in that the molar ratio of second ligand to palladium is at least 1.

7. A process as claimed in any one of claims 1 to 6, characterized in that the one or more sterically hindering groups are selected from benzyl, isopropyl, isobutyl, secondary butyl, tertiary butyl and cyclohexyl groups.

8. A process as claimed in any one of claims 1 to 7, characterized in that any non-sterically hindering groups present are selected from methyl, ethyl, n-propyl, n-butyl, phenyl and chlorophenyl groups.

**Patentansprüche**

1. Verfahren zur Herstellung von 1,7-Octadien umfassend die Hydrodimerisierung von Butadien in Gegenwart einer katalytischen Menge Palladium und eines tertiären organischen Phosphorliganden,

Ameisensäure oder einem Salz davon und gegebenenfalls einem Lösungsmittel, dadurch gekennzeichnet, daß ein Gemisch von Liganden vorhanden ist, umfassend einen ersten Liganden ausgewählt aus der Gruppe bestehend aus tertiären organischen Phosphinen, Phosphiniten und Phosphoniten der Formel $(RO)_aPR_b$, in der a+b=3, a 0, 1 oder 2 ist und zumindest ein R eine sterisch gehinderte Gruppe bedeutet, die eine verzweigte Alkylgruppe oder eine Aralkyl-, Alkenyl- oder Cycloalkylgruppe ist, wobei die erwähnten Gruppen 3 bis 10 Kohlenstoffatome besitzen, während die Verzweigung in den Alkylgruppen an einem Kohlenstoffatom vorliegt, das sich nicht mehr als 2 Kohlenstoffatome von dem Phosphoratom entfernt befindet, und mindestens einen weiteren Liganden, ausgewählt aus der Gruppe bestehend aus tertiären organischen Phosphinen, Phosphiniten, Phosphoniten und Phosphiten der allgemeinen Formel $(RO)_{a'}PR_{b'}$, in der R eine organische Gruppe bedeutet und in der a'+b'=3, a' 0, 1, 2 oder 3 und a' nicht gleich a ist.

2. Verfahren nach Anspruch 1, dadurch gekennzeichnet, daß die organischen Gruppen Aryl-, Alkyl-, Alkenyl-, Cycloalkyl-, Aralkyl- oder Alkarylgruppen mit nicht mehr als 20 Kohlenstoffatomen sind.

3. Verfahren nach Anspruch 1, dadurch gekennzeichnet, daß eine der organischen Gruppen des ersten oder zweiten Liganden ein vernetztes synthetisches Harz ist.

4. Verfahren nach Anspruch 3, dadurch gekennzeichnet, daß das Harz ein vernetztes Polystyrol, Poly($\alpha$-alkyl)acrylat, Polycarbonat oder Polyamid ist.

5. Verfahren nach einem der Ansprüche 1 bis 4, dadurch gekennzeichnet, daß die Menge an Palladium 1 bis $10^{-6}$ Mol beträgt (molar ist).

6. Verfahren nach einem der Ansprüche 1 bis 5, dadurch gekennzeichnet, daß das Molverhältnis von zweitem Liganden zu Palladium mindestens 1 ist.

7. Verfahren nach einem der Ansprüche 1 bis 6, dadurch gekennzeichnet, daß die eine oder die sterisch gehinderten Gruppen ausgewählt ist (sind aus Benzyl, Isopropyl, Isobutyl, sek.-Butyl, tert.-Butyl und Cyclohexyl).

8. Verfahren nach einem der Ansprüche 1 bis 7, dadurch gekennzeichnet, daß etwaige, nicht sterisch hindernde Gruppen, die vorhanden sind, ausgewählt sind aus Methyl-, Ethyl-, n-Propyl-, n-Butyl-, Phenyl- und Chlorphenylgruppen.

## Revendications

1. Un procédé pour la préparation de 1,7-octadiène comprenant l'hydrodimérisation de butadiène en présence d'une quantité catalytique de palladium et d'un ligand organo-phosphore tertiaire, d'acide formique ou d'un sel de cet acide et éventuellement d'un solvant, caractérisé en ce qu'un mélange de ligands est présent comprenant un premier ligand choisi parmi les organo-phosphines, phosphinites et phosphonites tertiaires représentés par la formule $(RO)_aPR_b$, dans laquelle a+b=3, a est 0, 1 ou 2 et au moins un R est un groupe stériquement empêché qui est un groupe alcoyle ramifié ou un groupe aralcoyle, alcényle ou cycloalcoyle, les groupes mentionnés ci-dessus ayant de 3 à 10 atomes de carbone, tandis que la ramification présente dans les groupes alcoyle se trouve à une atome de carbone qui n'est pas séparé de l'atome de phosphore par plus de deux atomes de carbone, et au moins un autre ligand choisi parmi les organo-phosphines, phosphinites et phosphonites tertiaires représentés par la formule $(RO)_{a'}PR_{b'}$, dans laquelle R est un groupe organique et a'+b'=3, a' est 0, 1, 2 ou 3 et a' n'est pas égal à a.

2. Un procédé selon la revendication 1, caractérisé en ce que les groupes organiques sont des groupes aryle, alcoyle, alcényle, cycloalcoyle, aralcoyle ou alcaryle ne contenant pas plus de 20 atomes de carbone.

3. Un procédé selon la revendication 1, caractérisé en ce qu'un des groupes organiques dans le premier ligand ou le deuxième est une résine synthétique réticulée.

4. Un procédé selon la revendication 3, caractérisé en ce que la résine est du polystyrène, du poly(alpha-alcoyl)acrylate, du polycarbonate ou du polyamide réticulé.

5. Un procédé selon l'une quelconque des revendications 1 à 4, caractérisé en ce que la quantité molaire de palladium est comprise entre 1 et $10^{-6}$.

6. Un procédé selon l'une quelconque des revendications 1 à 5, caractérisé en ce que le rapport molaire du deuxième ligand au palladium est au moins 1.

7. Un procédé selon l'une quelconque des revendications 1 à 6, caractérisé en ce que le ou les groupes produisant un empêchement stérique sont choisis parmi les groupes benzyle, isopropyle, isobutyle, sec.-butyle, tert.-butyle et cyclohexyle.

8. Un procédé selon l'une quelconque des revendications 1 à 7, caractérisé en ce que tous groupes ne produisant pas d'empêchement stérique présents sont choisis parmi des groupes méthyle, éthyle, n-propyle, n-butyle, phényle et chlorophényle.